(19) Europäisches Patentamt / European Patent Office / Office européen des brevets

(11) **EP 3 006 048 A1**

(12) **EUROPEAN PATENT APPLICATION**
published in accordance with Art. 153(4) EPC

(43) Date of publication:
**13.04.2016 Bulletin 2016/15**

(21) Application number: **14803436.6**

(22) Date of filing: **30.05.2014**

(51) Int Cl.:
**A61K 47/38** (2006.01)    **A61K 9/20** (2006.01)
**A61K 47/12** (2006.01)    **A61K 47/26** (2006.01)
**A61K 47/32** (2006.01)    **A61K 47/34** (2006.01)
**A61K 47/36** (2006.01)    **A61K 47/46** (2006.01)

(86) International application number:
**PCT/JP2014/064415**

(87) International publication number:
**WO 2014/192918 (04.12.2014 Gazette 2014/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **31.05.2013 JP 2013115694**

(71) Applicant: **Hisamitsu Pharmaceutical Co., Inc.
Tosu-shi, Saga 841-0017 (JP)**

(72) Inventors:
• **YOSHINAGA, Takaaki
Tosu-shi
Saga 841-0017 (JP)**

• **NAGASE, Yuko
Tosu-shi
Saga 841-0017 (JP)**
• **NAKANISHI, Toshihiro
Tosu-shi
Saga 841-0017 (JP)**

(74) Representative: **Grünecker Patent- und
Rechtsanwälte
PartG mbB
Leopoldstraße 4
80802 München (DE)**

(54) **ORAL CAVITY PATCH**

(57)     An oral cavity patch comprising: a drug layer and an adhesive layer, wherein the drug layer comprises a drug-release-controlling agent consisting or at least one selected from the group consisting of a hydroxyalkyl alkyl cellulose, hydroxypropyl cellulose, a sodium carboxyalkyl cellulose, ethyl cellulose, hydrogenated castor oil, and a sucrose fatty acid ester.

## Description

## Technical Field

The present invention relates to an oral cavity patch.

## Background Art

As an oral cavity patch, which is attached to the oral mucosa, the followings are known for example: a slow-releasing medical preparation comprising an adhesive polymer layer and a non-adhesive layer, wherein a drug is present in the non-adhesive layer (Patent literature 1); a patch for the oral mucosa comprising a pressure-sensitive adhesive layer and a drug-containing layer, wherein the drug-containing layer includes a nicotine or a salt thereof (Patent literature 2); and a solid preparation having a layer to be contacted with the mucous and comprising a pressure-sensitive adhesive, wherein the layer to be contacted with the mucous has a convex shape in its central vertical cross section, and the preparation can be provided with a layer not to be contacted with the mucous which supports the layer to be contacted with the mucous and intensively contains an active ingredient (Patent literature 3).

## Citation List

## Patent Literature

Patent Literature 1: Japanese Unexamined Patent Publication No. S55-62012
Patent Literature 2: Japanese Unexamined Patent Publication No. H3-209326
Patent Literature 3: Japanese Unexamined Patent Publication No. 2006-96728

## Summary of Invention

## Technical Problem

However, when any one of these oral cavity patches is attached to the oral mucosa, the dissolution of the drug from the drug-containing layer (drug layer) completes in a short time, or the drug layer breaks down in a short time, which is problematic.
Then, an object of the present invention is to provide an oral cavity patch in which the dissolution of the drug from the drug layer or the breakdown of the drug layer is delayed to a sufficient level for practical use.

## Solution to Problem

The present invention provides an oral cavity patch comprising a drug layer and an adhesive layer, wherein the drug layer comprises a drug-release-controlling agent consisting of at least one selected from the group consisting of a hydroxyalkyl alkyl cellulose, hydroxypropyl cellulose, a sodium carboxyalkyl cellulose, ethyl cellulose, hydrogenated castor oil, and a sucrose fatty acid ester. Because the oral cavity patch of the present invention has the constitution described above, the dissolution of the drug from the drug layer or the breakdown of the drug layer is delayed to a sufficient level for practical use.
Here, the drug-release-controlling agent of the present invention means an ingredient which delays the dissolution of the drug from the drug layer (layer containing the drug) or the breakdown of the drug layer, and the adhesive layer of the present invention means a layer having an area to adhere to the oral mucosa.
As the hydroxyalkyl alkyl cellulose, hydroxypropyl methyl cellulose is preferable. As hydroxypropyl methyl cellulose, one having a methoxy group (%) (methoxy content in %) of 19 to 30% and a hydroxypropoxy group (%) (hydroxypropoxy content in %) of 4 to 12% can be employed. As hydroxypropyl methyl cellulose, one satisfying the following conditions can be also employed: (1) a methoxy cgroup (%) of 27 to 30% and hydroxypropoxy group (%) of 4 to 7.5%; (2) a methoxy group (%) of 19 to 24% and a hydroxypropoxy group (%) of 4 to 12%; or (3) a methoxy group (%) of 28 to 30% and a hydroxypropoxy group (%) of 7 to 12%. Among these conditions (1) to (3), the condition (1) or (2) is preferable. By employing the above-mentioned hydroxyalkyl alkyl cellulose, the dissolution of the drug from the drug layer can be delayed greatly. Each of the methoxy group (%) and the hydroxypropoxy group (%) means a value obtained according to the quantitative method described in the item "Hypromellose" in The Japanese Pharmacopoeia, Sixteenth Edition.

As hydroxypropyl cellulose, one capable of providing a 2% aqueous solution having a viscosity of 6.0 to 10 mPa·s at 20°C is preferable. Hydroxypropyl cellulose exhibiting such a viscosity is suitable for delaying the dissolution of the drug from the drug layer. The viscosity means a value obtained for an aqueous solution of hydroxypropyl cellulose, according to "Viscosity Determination, Method II Viscosity measurement by rotational viscometer" in the chapter of General Test, Process and Apparatus in The Japanese Pharmacopoeia, Sixteenth Edition.

As the sucrose fatty acid ester, a sucrose fatty acid ester having a behenic acid residue as a fatty acid residue and an HLB value of 3, or a sucrose fatty acid ester having a stearic acid residue as a fatty acid residue and an HLB value of 11 is suitable for delaying the dissolution of the drug from the drug layer more.

The adhesiveness to the oral mucosa is improved, when the adhesive layer comprises, as an adhesive agent, at least one selected from the group consisting of a carboxyalkyl cellulose, a carboxyalkyl cellulose salt, alginic acid, an alginate salt, poly(N-vinyl lactam), polyvinyl alcohol, hydroxypropyl cellulose, carboxyvinyl polymer, pullulan, and pregelatinized starch.

It is possible to incorporate (1) a carboxyalkyl cellulose or a carboxyalkyl cellulose salt, (2) a carboxyalkyl cellulose or a carboxyalkyl cellulose salt, plus poly(N-vinyl lactam), (3) a carboxyalkyl cellulose or a carboxyalkyl cellulose salt, plus polyvinylpyrrolidone, as an adhesive agent into the oral cavity patch. When the ingredient(s) described above are used as the adhesive agent, not only the dissolution of the drug from the drug layer or the breakdown of the drug layer can be delayed, but also the adhesiveness to the oral mucosa becomes especially excellent.

When the oral cavity patch of the present invention is a tablet, it is easy to handle and excellent in portability.

**Advantageous Effects of Invention**

A oral cavity patch in which the dissolution of the drug from the drug layer or the breakdown of the drug layer is delayed to a sufficient level for practical use is provided.

**Description of Embodiments**

The oral cavity patch according to the embodiment comprises a drug layer and an adhesive layer. First, ingredients contained in the drug layer will be described in detail.

The drug layer comprises a drug-release-controlling agent consisting of at least one selected from the group consisting of a hydroxyalkyl alkyl cellulose, hydroxypropyl cellulose, a sodium carboxyalkyl cellulose, ethyl cellulose, hydrogenated castor oil, and a sucrose fatty acid ester.

The hydroxyalkyl alkyl cellulose is obtained by replacing some of hydrogen atoms of hydroxy groups (OH groups) in cellulose with alkyl groups and hydroxyalkyl groups. The alkyl group of the hydroxyalkyl moiety in the hydroxyalkyl alkyl cellulose preferably has 1 to 6 carbon atoms, and more preferably has 1 to 3 carbon atoms. The alkyl group constituting the alkyl cellulose moiety in the hydroxyalkyl alkyl cellulose preferably has 1 to 6 carbon atoms, and more preferably has 1 to 3 carbon atoms. The hydroxyalkyl alkyl celluloses may be used singly or in combinations of two or more. Hydroxypropyl methyl cellulose is particularly suitably used as the hydroxyalkyl alkyl cellulose.

The methoxy group (%) (methoxy content in %) and the hydroxypropoxy group (%) (hydroxypropoxy content in %) of hydroxypropyl methyl cellulose can be obtained according to the quantitative method described in the item "Hypromellose" in The Japanese Pharmacopoeia, Sixteenth Edition. The methoxy group (%) and the hydroxypropoxy group (%) of hydroxypropyl methyl cellulose is preferably 19 to 30% and 4 to 12% according to this quantitative method, respectively. Also, preferred are hydroxypropyl methyl cellulose having a methoxy group (%) of 27 to 30% and a hydroxypropoxy group (%) of 4 to 7.5%, and/or hydroxypropyl methyl cellulose having a methoxy group (%) of 19 to 24% and a hydroxypropoxy group (%) of 4 to 12%. Further, hydroxypropyl methyl cellulose having a methoxy group (%) of 28 to 30% and a hydroxypropoxy group (%) of 7 to 12% is also preferable. By using such a hydroxypropyl methyl cellulose, the dissolution of the drug from the drug layer can be delayed greatly.

As hydroxypropyl methyl cellulose, one capable of providing a 2% aqueous solution having a viscosity of 2 to 150000 mPa·s at 20°C can be used. Here, the value of the viscosity can be obtained according to the viscosity determination method described in the item "Hypromellose" in The Japanese Pharmacopoeia, Sixteenth Edition. It is preferable to use as hydroxypropyl methyl cellulose at least one capable of providing a 2% aqueous solution having any one of the following viscosity at 20°C: (1) one having a viscosity of 2.5 to 3.5 mPa·s, (2) one having a viscosity of 80 to 120 mPa·s, (3) one having a viscosity of 3000 to 5600 mPa·s, (4) one having a viscosity of 11250 to 21000 mPa·s, and (5) one having a viscosity of 75000 to 140000 mPa·s. By using such a hydroxypropyl methyl cellulose, the dissolution of the drug from the drug layer can be delayed greatly.

As hydroxypropyl cellulose, one capable of providing an aqueous solution having a lower viscosity is preferable, and for example, one capable of providing a 2% aqueous solution having a viscosity of 100 mPa·s or less, particularly 6.0 to 10 mPa·s, at 20°C is preferable. Here, as mentioned above, the viscosity is measured according to "Viscosity Determination, Method II Viscosity measurement by rotational viscometer" in the chapter of General Test, Process and Apparatus in The Japanese Pharmacopoeia, Sixteenth Edition.

Examples of hydroxypropyl cellulose include an ultrafine particle grade (a hydroxypropyl cellulose particle having a median diameter D50 of 20 $\mu$m or passing through 330 mesh), a fine particle grade (a hydroxypropyl cellulose particle having a median diameter D50 of 80 to 110 $\mu$m or passing through 100 mesh), and a normal grade (a hydroxypropyl cellulose particle having a median diameter D50 of 85 to 185 $\mu$m or passing through 40 mesh). When the oral cavity patch is formulated as a tablet, an ultrafine particle grade or a fine particle grade is preferably used because the hardness of the tablet becomes high. When the hardness of the tablet is high, fracture is difficult to occur.

Carboxymethyl cellulose (Carmellose) sodium can be suitably used as the sodium carboxyalkyl cellulose. Particularly, one having a degree of etherification of 0.6 to 1.5 is preferable, and one capable of providing a 1% aqueous solution having a viscosity of 10 to 4500 mPa·s at 25°C is also preferable. This viscosity means a value obtained by dissolving sodium carboxyalkyl cellulose such as sodium carboxymethyl cellulose in water and then measuring the viscosity of the resulting solution with a Brookfield viscometer under the condition of the liquid temperature of 25°C.

Ethyl cellulose, hydrogenated castor oil, and the sucrose fatty acid ester are not limited, but as the sucrose fatty acid ester, one having a fatty acid residue having 12 or more carbon atoms and an HLB of 1 to 15 is appropriate. Particularly, a sucrose fatty acid ester having a behenic acid residue as a fatty acid residue and an HLB value of 3, or a sucrose fatty acid ester having a stearic acid residue as a fatty acid residue and an HLB value of 11 is preferable.

The amount of the drug-release-controlling agent formulated can be an amount capable of delaying the dissolution of the drug from the drug layer or the breakdown of the drug layer, but the content of the drug-release-controlling agent is preferably 10% by mass or more, particularly preferably 15% by mass or more based on the total mass of the ingredients of the drug layer.

As the drug to be contained in the drug layer, an oral bactericide, a breath deodorizer (deodorant), an anti-inflammatory drug, a periodontal disease therapeutic agent, etc. can be used. When the drug is nicotine or a salt thereof, the oral cavity patch can be used for smoking cessation, the treatment for nicotine dependence, or the like.

Examples of the oral bactericide include cetylpyridinium chloride, chlorhexidine hydrochloride, benzethonium chloride, benzalkonium chloride, iodine, hinokitiol, and lysozyme chloride.

Examples of the breath deodorizer (deodorant) include a chlorophyllin such as sodium copper chlorophyllin, a polyphenol, and a flavonoid.

Examples of the anti-inflammatory drug include glycyrrhetinic acid or a salt thereof, indometacin, azulene, hydrocortisone, prednisolone, dexamethasone, and triamcinolone acetonide.

Examples of the periodontal disease therapeutic agent include azithromycin, cefalexin, and levofloxacin.

In addition to the ingredients mentioned above, the drug layer may comprise any of ingredients including a pH regulator, a cooling agent such as 1-menthol, a sweetener, a flavoring agent, a colorant, an absorbent, a stabilizer, and an aromatizing agent (fragrance).

Next, the ingredients contained in the adhesive layer will be described. The adhesive layer usually comprises an adhesive agent as an ingredient for adhering to the oral mucosa. As the adhesive agent, preferred is at least one selected from the group consisting of a carboxyalkyl cellulose, a carboxyalkyl cellulose salt, alginic acid, an alginate salt, poly(N-vinyl lactam), polyvinyl alcohol, hydroxypropyl cellulose, a carboxyvinyl polymer, pullulan, and pregelatinized starch.

As the adhesive agent, a carboxyalkyl cellulose or a carboxyalkyl cellulose salt is preferred, and examples of the carboxyalkyl cellulose salt include sodium carboxymethyl cellulose. As the adhesive agent, a carboxyalkyl cellulose or a carboxyalkyl cellulose salt may be used in combination with an ingredient of the adhesive agent mentioned above other than these. As the ingredient of the adhesive agent for combination use, alginic acid, an alginate salt, poly(N-vinyl lactam), polyvinyl alcohol, hydroxypropyl cellulose, pullulan, or the like is preferable. Among these, poly(N-vinyl lactam) is preferable, and particularly polyvinylpyrrolidone (povidone) is preferable in terms of adhesiveness.

The content of the adhesive agent is preferably 25 to 75% by mass, particularly preferably 40 to 60% by mass based on the total mass of the ingredients of the adhesive layer.

The oral cavity patch comprising such a drug layer and an adhesive layer has no limitation on the dosage form, the shape and the size of the drug layer and the adhesive layer, and the like, and further may comprises a component other than the drug layer and the adhesive layer, as long as the dissolution of the drug from the drug layer or the breakdown of the drug layer can be delayed while also satisfying the condition that the adhesive layer can adhere to the oral mucosa.

Preferable example of the dosage form includes a tablet, a sheet, and a film, and a tablet is particularly preferable because it is easy to handle and excellent in portability.

When the oral cavity patch is a tablet, a bilayer tablet (laminated tablet), which consists of two layers, i.e., the drug layer and the adhesive layer, is suitable. Although the shape and the size of the tablet are designed taking easiness in attachment, difficulty in fracture and the like into consideration, the diameter of the tablet is, for example, 0.5 to 1.5 cm, and preferably 0.7 to 1.0 cm, and the thickness of the tablet is, for example, 1.0 to 2.5 mm, and preferably 1.5 to 2.0 mm. The weight of the tablet can be, for example, 50 to 200 mg, and preferably 100 to 150 mg. In the weight of the tablet, the weight of the drug layer can be, for example, 30 to 150 mg, and preferably 50 to 100 mg, and the weight of the adhesive layer can be, for example, 10 to 80 mg, and preferably 30 to 60 mg.

The drug layer and the adhesive layer of the tablet may further comprise an ingredient commonly used in a tablet, including an excipient such as lactose hydrate, a binder such as a crystalline cellulose, and a lubricant such as a stearate salt and talc.

Example of the lactose hydrate includes a fine particle grade (a fine particle of lactose hydrate) and a granulate grade (a granule of lactose hydrate). When the oral cavity patch is a tablet, the granule of lactose hydrate is preferably used, because the hardness of the tablet becomes high. When the hardness of the tablet is high, fracture is difficult to occur. As the granule of lactose hydrate, one having an average particle size of 100 to 500 $\mu$m is preferable,

In a case where the oral cavity patch is provided as a tablet, it is preferable to make the hardness thereof high. When the hardness of the tablet is high, fracture of the tablet can less occur. The hardness of the tablet can be measured with a tablet durometer, and the hardness measured is preferably 10 N or more, and more preferably 20 N or more.

In a case where the oral cavity patch is provided as a tablet having a high hardness, the thickness of the whole is preferably from 1.5 to 2 mm. By the thickness of the tablet within such a range, the effect when the tablet has high hardness described above can be obtained.

The oral cavity patch can be produced according to the following method. In a case where the oral cavity patch is a tablet, for example, each of the ingredients of the drug layer is weighed, and mixed together to form a powder for tableting, followed by subjecting it to tableting; and then, another powder for tableting obtained by weighing each of the ingredients of the adhesive layer and mixing them together is added thereto, followed by tableting, to thereby produce a tablet. A common compression molding machine, a common bilayer tableting machine, or the like can be used for tableting. The oral cavity patch in another dosage form such as a sheet or a film can be also produced by laminating a drug layer and an adhesive layer according to a conventional method.

When the oral cavity patch is used, the adhesive layer side thereof is pressed on the oral mucosa to adhere thereto. Once the oral cavity patch adheres, then the release of the drug from the drug layer starts. The time until the dissolution of the drug from the drug layer completes or the drug layer breaks is usually 20 minutes or more and 180 minutes or less, and particularly 30 minutes or more and 60 minutes or less. The number of the oral cavity patch used per day should be a number which brings the expected efficacy of the oral cavity patch, and cannot be generalized, but one oral cavity patch is usually used at a time.

**Examples**

Test Examples 1 to 11 and Comparative Test Examples 1 to 15

Nicotine was weighed into a container, and a stabilizer and 1-menthol were added thereto, then nicotine was dissolved. This was added to hydrous silicon dioxide and mixed lightly, and then the resultant was transferred to a mixer and mixed. Lactose hydrate, crystalline cellulose, a pH regulator, Food Yellow No. 5, and sucralose were added thereto and mixed. The resultant powder was discharged into a bag, and magnesium stearate and talc were added thereto and mixed by hand. The ingredient A (drug-release-controlling agent) shown in Table 2 was further added thereto and mixed by hand to obtain a powder for tableting. The powder for tableting was weighed, and subjected to tableting with a compression molding machine to produce a monolayer tablet (diameter: 10 mm) of a drug layer weighing 80 mg which has no adhesive layer. The percentage (% by mass) of each ingredient in the formulation of this monolayer tablet is shown in Table 1.

The produced monolayer tablet as a specimen was tested for the dissolution rate (%) after 10 minutes with a dissolution tester. This dissolution test was conducted according to 6.10 Dilution Test (3.1. Basket Method or Paddle Method) in The Japanese Pharmacopoeia, Sixteenth Edition, and a dissolution tester NTR-6100 manufactured by Toyama Sangyo Co. Ltd. was used as a dissolution tester. The specific test method was as follows. 300 mL of diluted McIlvaine buffer solution (pH 6.0) was placed in a vessel and kept at a temperature of $37\pm0.5°C$. One sample was attached to the predetermined position of the paddle and submerged into the test liquid, and the test was started. The number of revolutions of the paddle was set to 50 rpm, and 3 mL of the dissolution medium was collected after 10 minutes. The dissolution medium collected was filtered by a membrane filter having the pore size of 0.45 $\mu$m, and then the drug concentration in the resultant dissolution medium was measured with high performance liquid chromatography, followed by calculating the dissolution rate.

$$\text{Dissolution rate after 10 minutes (\%)} = C_T \times 300 \times (1/M) \times 100$$

M: amount of nicotine per tablet indicated (mg)

$C_T$: concentration in sample solution (mg/mL) = $C_S \times (A_T/A_S)$

Cs: concentration in standard solution (mg/mL)

$A_T$: peak area of nicotine of sample solution

As: peak area of nicotine of standard solution

Ingredients A and the dissolution rates (%) in Test Examples 1 to 11 and Comparative Test Examples 1 to 15 are shown in Table 2. It was revealed by this test that hydroxypropyl cellulose, hydroxypropyl methyl cellulose, Carmellose sodium, ethyl cellulose, hydrogenated castor oil, and a sucrose fatty acid ester are excellent as a drug-release-controlling agent.

[Table 1]

| Name of Ingredient | Percentage in Formulation [% by mass] |
| --- | --- |
| Nicotine | 2.5 |
| Lactose Hydrate | 49.7 |
| Crystalline Cellulose | 14.7 |
| Ingredient A | 20.0 |
| Hydrous Silicon Dioxide | 5.0 |
| Magnesium Stearate | 0.5 |
| Talc | 0.5 |
| Stabilizer | 0.2 |
| pH Regulator | 2.8 |
| Food Yellow No. 5 | 0.1 |
| l-Menthol | 2.0 |
| Sucralose | 2.0 |

[Table 2]

| | | Ingredient A | Dissolution Rate [%] |
| --- | --- | --- | --- |
| Test Example 1 | | Hydroxypropyl Cellulose (Viscosity of 2% Aqueous Solution at 20°C: 6.0 to 10.0 mPa·s) | 47 |
| Test Example 2 | | Hypromellose 2208 (Viscosity of 2% Aqueous Solution at 20°C: 80 to 120 mPa·s, Methoxy Group (%): 19 to 24%, Hydroxypropoxy group (%): 4 to 12%) | 36 |
| Test Example 3 | | Hypromellose 2208 (Viscosity of 2% Aqueous Solution at 20°C: 3000 to 5600 mPa·s, Methoxy Group (%): 19 to 24%, Hydroxypropoxy group (%): 4 to 12%) | 27 |
| Test Example 4 | | Hypmmellose 2208 (Viscosity of 2% Aqueous Solution at 20°C:11250 to 21000 mPa·s, Methoxy Group (%) : 19 to 24%, Hydroxypropoxy group (%): 4 to 12%) | 22 |
| Test Example 5 | | Hypromellose 2208 (Viscosity of 2% Aqueous Solution at 20°C: 75000 to 140000 mPa·s, Methoxy Group (%): 19 to 24%, Hydroxypropoxy group (%): 4 to 12%) | 21 |
| Test Example 6 | | Carmellose Sodium (Degree of Etherification: 0.8 to 1.0, Viscosity of 1 % Aqueous Solution at 25°C: 10 to 20 mPa·s) | 32 |

(continued)

| | Ingredient A | Dissolution Rate [%] |
|---|---|---|
| Test Example 7 | Ethyl Cellulose | 36 |
| Test Example 8 | Hydrogenated Oil (Hydrogenated Castor Oil) | 30 |
| Test Example 9 | Sucrose Fatty Acid Ester (Fatty Acid: Behenic Acid, HLB Value: 3) | 36 |
| Test Example 10 | Sucrose Fatty Acid Ester (Fatty Acid: Stearic Acid, HLB Value:11) | 28 |
| Test Example 11 | Sucrose Fatty Acid Ester (Fatty Acid: Stearic Acid, HLB Value: 3) | 45 |
| Comparative Test Example 1 | Methyl Cellulose | 89 |
| Comparative Test Example 2 | Carmellose | 101 |
| Comparative Test Example 3 | Carmellose Calcium | 104 |
| Comparative Test Example 4 | Croscarmellose Sodium | 103 |
| Comparative Test Example 5 | Povidone (K-Value: 30) | 103 |
| Comparative Test Example 6 | Crospovidone | 88 |
| Comparative Test Example 7 | Polyvinyl Alcohol | 99 |
| Comparative Test Example 8 | Pregelatinized starch (Degree of Gelatinization: 90 to 100%, Average Particle Size: 20 to 40 ($\mu$m) | 100 |
| Comparative Test Example 9 | Pregelatinized starch (Degree of Gelatinization: 70 to 80%, Average Particle Size: 40 to 60 $\mu$m) | 106 |
| Comparative Test Example 10 | PregelatInized starch (Degree of Gelatinization: 90 to 100%, Average Particle Size: 70 to 90 $\mu$m) | 97 |
| Comparative Test Example 11 | Partially Pregelatinized starch | 100 |
| Comparative Test Example 12 | Sodium Starch Glycolate | 91 |
| Comparative Test Example 13 | Aminoalkyl Methacrylate Copolymer RS (Ethyl Acrylate : Methyl Metacrylate : Trimethylammoniumethylmethacrylic Chloride =1 : 2 : 0.2) | 87 |
| Comparative Test Example 14 | Aminoalkyl Methacrylate Copolymer RS (Ethyl Acrylate : Methyl Metacrylate : Trimethylammoniumethylmethacrylic Chloride =1:2: 0.1) | 82 |
| Comparative Test Example 15 | Hydrogenated Oil (Hydrogenated Rapeseed Oil) | 89 |

Examples 1 to 6

Nicotine was weighed into a container, and a stabilizer and 1-menthol were added thereto, then nicotine was dissolved. This was added to hydrous silicon dioxide and mixed lightly, and then the resultant mixture was transferred to a mixer and mixed. Lactose hydrate, crystalline cellulose, a pH regulator, Food Yellow No. 5, and sucralose were added thereto and mixed. The resultant powder was discharged into a bag, and magnesium stearate and talc were added thereto and mixed by hand. The ingredient B (drug-release-controlling agent) shown in Table 3 was further added thereto and mixed by hand to obtain a drug layer powder for tableting. In a container, lactose hydrate, crystalline cellulose, and Carmellose sodium were placed and mixed by hand, and further magnesium stearate and talc were added thereto and mixed by hand to obtain an adhesive layer powder for tableting. With a single punch tableting machine for bilayer tablets, the drug layer powder for tableting was first tableted lightly, and the adhesive layer powder for tableting was then added thereto and strongly tableted to produce a bilayer tablet (diameter: 9 mm) having a drug layer weighing 80 mg + an adhesive layer weighing 40 mg. The percentage (% by mass) of each ingredient in the formulation of the drug layer is shown in Table 4. The percentage (% by mass) of each ingredient in the formulation of the adhesive layer is shown in Table 5.

The produced bilayer tablet as a specimen was tested for the dissolution rate (%) after 10 minutes with a dissolution tester in the same manner as in Test Example 1 etc. mentioned above. Ingredients B and the dissolution rates (%) in Examples 1 to 6 are shown in Table 3.

[Table 3]

| | | Ingredient B | Dissolution Rate [%] |
|---|---|---|---|
| | Example 1 | Hypromellose 2208 (Viscosity of 2% Aqueous Solution at 20°C: 75000 to 140000 mPa·s, Methoxy Group (%): 19 to 24%, Hydroxypropoxy group (%): 4 to 12%) | 18 |
| | Example 2 | Hypromellose 2906 (Viscosity of 2% Aqueous Solution at 20°C: 3000 to 5600 mPa·s, Methoxy Group (%): 27 to 30%, Hydroxypropoxy group (%): 4 to 7.5%) | 18 |
| | Example 3 | Carmellose Sodium (Degree of Etherification: 0.7 to 0.9, Viscosity of 2% Aqueous Solution at 25°C: 1000 to 1400 mPa·s) | 15 |
| | Example 4 | Hypromellose 2208 (Viscosity of 2% Aqueous Solution at 20°C: 80 to 120 mPa·s, Methoxy Group (%): 19 to 24%, Hydroxypropoxy group (%): 4 to 12%) | 28 |
| | Example 5 | Hypromellose 2910 (Viscosity of 2% Aqueous Solution at 20°C: 7500 to 14000 mPa·s, Methoxy Group (%): 28 to 30%, Hydroxypropoxy group (%): 7 to 12%) | 40 |
| | Example 6 | Hypromellose 2910 (Viscosity of 2% Aqueous Solution at 20°C: 2.5 to 3.5 mPa·s, Methoxy Group (%): 28 to 30%, Hydroxypropoxy group (%): 7 to 12%) | 35 |

[Table 4]

| Name of Ingredient | Percentage in Formulation [% by mass] |
|---|---|
| Nicotine | 2.5 |
| Lactose Hydrate | 49.7 |
| Crystalline Cellulose | 14.7 |
| Ingredient B | 20.0 |
| Hydrous Silicon Dioxide | 5.0 |
| Magnesium Stearate | 0.5 |
| Talc | 0.5 |
| Stabilizer | 0.2 |
| pH Regulator | 2.8 |
| Food Yellow No. 5 | 0.1 |
| l-Menthol | 2.0 |
| Sucralose | 2.0 |

[Table 5]

| Name of Ingredient | Percentage in Formulation [% by mass] |
|---|---|
| Lactose Hydrate | 29.0 |
| Crystalline Cellulose | 20.0 |
| Carmellose Sodium (Degree of Etherification: 0.8 to 1.0, Viscosity of 1% Aqueous Solution at 25°C: 10 to 20 mPa·s) | 50.0 |
| Magnesium Stearate | 0.5 |
| Talc | 0.5 |

Examples 7 to 9

Nicotine was weighed into a container, and a stabilizer and 1-menthol were added thereto, then nicotine was dissolved. This was added to hydrous silicon dioxide and mixed lightly, and then the resultant was transferred to a mixer and mixed. Lactose hydrate, crystalline cellulose, Hypromellose, a pH regulator, Food Yellow No. 5, and sucralose were added thereto and mixed. The resultant powder was discharged into a bag, and magnesium stearate and talc were added thereto and mixed by hand to obtain a drug layer powder for tableting. Lactose hydrate, Carmellose sodium, and sodium alginate were weighed into a container, and mixed by hand to obtain an adhesive layer powder for tableting. With a compression molding machine, the drug layer powder for tableting was first compressed lightly, and the adhesive layer powder for tableting was then added thereto and strongly compressed for tableting to produce a bilayer tablet (diameter: 8 mm) having a drug layer weighing 51.2 mg + an adhesive layer weighing 51.2 mg. The percentage (% by mass) in each of the formulations of the drug layer and the adhesive layer in this bilayer tablet is shown in Table 6.

The produced bilayer tablet as a specimen was tested for the dissolution rate (%) after 10 minutes with a dissolution tester in the same manner as in Test Example 1 etc. mentioned above. The dissolution rates (%) in Examples 7 to 9 are shown in Table 6.

[Table 6]

| | Name of Ingredient | Percentage in Formulation [% by mass] | | |
|---|---|---|---|---|
| | | Example 7 | Example 8 | Example 9 |
| Drug Layer | Nicotine | 2.5 | 2.5 | 2.5 |
| | Lactose Hydrate | 59.7 | 54.7 | 49.7 |
| | Crystalline Cellulose | 14.7 | 14.7 | 14.7 |
| | Hypromellose 2208 (Viscosity of 2% Aqueous Solution at 20°C: 80 to 120 mPa·s) | 10.0 | 15.0 | 20.0 |
| | Hydrous Silicon Dioxide | 5.0 | 5.0 | 5.0 |
| | Magnesium Stearate | 0.5 | 0.5 | 0.5 |
| | Talc | 0.5 | 0.5 | 0.5 |
| | Stabilizer | 0.2 | 0.2 | 0.2 |
| | pH Regulator | 2.8 | 2.8 | 2.8 |
| | Food Yellow No. 5 | 0.1 | 0.1 | 0.1 |
| | l-Menthol | 2.0 | 2.0 | 2.0 |
| | Sucralose | 2.0 | 2.0 | 2.0 |

(continued)

| | Name of Ingredient | Percentage in Formulation [% by mass] | | |
|---|---|---|---|---|
| | | Example 7 | Example 8 | Example 9 |
| Adhe sive layer | Lactose Hydrate | 50.0 | 50.0 | 50.0 |
| | Carmellose Sodium (Degree of Etherification: 0.8 to 1.0, Viscosity of 1% Aqueous Solution at 25°C: 10 to 20 mPa·s) | 25.0 | 25.0 | 25.0 |
| | Sodium Alginate | 25.0 | 25.0 | 25.0 |
| Dissolution Rate [%] | | 44 | 28 | 24 |

Examples 10 to 16 and Comparative Examples 1 to 3

Lactose hydrate, crystalline cellulose, hydroxypropyl cellulose, 1-menthol, saccharin sodium hydrate, sodium copper chlorophyllin, Blue No.1 aluminum lake, and a fragrance were placed in a mixer and mixed. Then, the resultant powder was discharged into a bag, and magnesium stearate and talc were added thereto and mixed by hand to obtain a drug layer powder for tableting. Ingredients of the adhesive layer were weighted into a container and mixed by hand to obtain an adhesive layer powder for tableting. With a compression molding machine, the drug layer powder for tableting was first compressed lightly, and the adhesive layer powder for tableting was then added thereto and strongly compressed for tableting to produce a bilayer tablet (diameter: 9 mm) having a drug layer weighing 80 mg + an adhesive layer weighing 40 mg. The percentage (% by mass) in the formulation of the drug layer and the percentage (% by mass) in the formulation of the adhesive layer in this bilayer tablet are shown in Tables 7 and 8, respectively.

An organoleptic test for the adhesiveness was conducted using the produced bilayer tablet as a specimen. The adhesive layer side of the bilayer tablet was pressed on the upper jaw of five adults each to adhere thereto, and the adhesiveness when licked was evaluated. The score was given according to the following evaluation criteria, and the average value (average score) was calculated. The grater the score is, the better the adhesiveness is.

Score 3: The bilayer tablet did not move.
Score 2: The bilayer tablet moved a little.
Score 1: The bilayer tablet moved.
Score 0: The bilayer tablet fell down.

Ingredients C and the average scores in Examples 10 to 16 and Comparative Examples 1 to 3 are shown in Table 9.

[Table 7]

| Name of Ingredient | Percentage in Formulation [% by mass] |
|---|---|
| Lactose Hydrate | 57.0 |
| Crystalline Cellulose | 18.2 |
| Hydroxypropyl Cellulose (Viscosity of 2% Aqueous Solution at 20°C: 6.0 to 10.0 mPa·s) | 20.0 |
| Magnesium Stearate | 0.5 |
| Talc | 0.5 |
| I-Menthol | 1.5 |
| Saccharin Sodium Hydrate | 2.0 |
| Sodium Copper Chlorophyllin | 0.1 |
| Blue No.1 Aluminum Lake | 0.1 |
| Fragrance | 0.1 |

[Table 8]

| Name of Ingredient | Percentage in Formulation [% by mass] |
|---|---|
| Lactose Hydrate | 29.0 |
| Crystalline Cellulose | 20.0 |
| Carmellose Sodium (Degree of Etherification: 0.8 to 1.0, Viscosity of 1% Aqueous Solution at 25°C:10 to 20 mPa·s) | 29.0 |
| Magnesium Stearate | 0.5 |
| Talc | 0.5 |
| Ingredient C | 21.0 |

[Table 9]

| | Ingredient C | Average Score |
|---|---|---|
| Example 10 | Sodium Alginate | 2.0 |
| Example 11 | Povidone (K-Value: 90) | 3.0 |
| Example 12 | Polyvinyl Alcohol (Partially Saponified Product) | 2.2 |
| Example 13 | Hydroxypropyl Cellulose (Viscosity of 2% Aqueous Solution at 20C: 150 to 400 mPa·s) | 2.2 |
| Example 14 | Carboxyvinyl Polymer | 2.4 |
| Example 15 | Pullulan | 2.4 |
| Example 16 | Pregelatinized starch (Degree of Gelatinization: 90 to 100%, Average Particle Size: 20 to 40 $\mu$m) | 2.6 |
| Comparative Example 1 | Sodium Polyacrylate | 1.6 |
| Comparative Example 2 | Hypromellose 2910 (Viscosity of 2% Aqueous Solution at 20°C: 40 to 60 mPa·s) | 1.8 |
| Comparative Example 3 | Hydroxyethyl Cellulose | 1.6 |

Example 17

Ingredients of the adhesive layer were weighted into a container and mixed by hand to obtain an adhesive layer powder for tableting. With a compression molding machine, a drug layer powder for tableting was first compressed lightly, and the adhesive layer powder for tableting was then added thereto and strongly compressed for tableting to produce a bilayer tablet (diameter: 9 mm) having a drug layer weighing 80 mg + an adhesive layer weighing 40 mg. The percentage (% by mass) of the ingredient in the formulation of the drug layer in this bilayer tablet is shown in Table 10. The percentage (% by mass) of the ingredient in the formulation of the adhesive layer is the same as that in Example 11.

[Table 10]

| Name of Ingredient | Percentage in Formulation [% by mass] |
|---|---|
| Cetylpyridinium Chloride | 1.25 |
| Hydroxypropyl Cellulose | 20.0 |
| Crystalline Cellulose | 18.0 |

(continued)

| Name of Ingredient | Percentage in Formulation [% by mass] |
|---|---|
| Lactose Hydrate | 56.0 |
| Other (Fragrance, Breath Deodorizer, Cooling Agent, Sweetener, Colorant, etc.) | 4.75 |

**Industrial Applicability**

The oral cavity patch of the present invention can be utilized as an oral cavity patch in which the dissolution of the drug from the drug layer or the breakdown of the drug layer can be delayed.

**Claims**

1. An oral cavity patch comprising:

    a drug layer; and
    an adhesive layer,
    wherein the drug layer comprises a drug-release-controlling agent consisting of at least one selected from the group consisting of a hydroxyalkyl alkyl cellulose, hydroxypropyl cellulose, a sodium carboxyalkyl cellulose, ethyl cellulose, hydrogenated castor oil, and a sucrose fatty acid ester.

2. The oral cavity patch according to claim 1, wherein the hydroxyalkyl alkyl cellulose is hydroxypropyl methyl cellulose.

3. The oral cavity patch according to claim 2, wherein hydroxypropyl methyl cellulose has
   a methoxy group (%) of 19 to 30% and a hydroxypropoxy group (%) of 4 to 12%.

4. The oral cavity patch according to claim 2 or 3, wherein hydroxypropyl methyl cellulose has
   a methoxy group (%) of 27 to 30% and a hydroxypropoxy group (%) of 4 to 7.5%; a methoxy group (%) of 19 to 24% and a hydroxypropoxy group (%) of 4 to 12%; or a methoxy group (%) of 28 to 30% and a hydroxypropoxy group (%) of 7 to 12.

5. The oral cavity patch according to any one of claims 1 to 4, wherein a viscosity of a 2% aqueous solution of the hydroxypropyl cellulose at 20°C is 6.0 to 10 mPa·s.

6. The oral cavity patch according to any one of claims 1 to 5, wherein the sucrose fatty acid ester is a sucrose fatty acid ester having a behenic acid residue as a fatty acid residue and an HLB value of 3, or a sucrose fatty acid ester having a stearic acid residue as a fatty acid residue and an HLB value of 11.

7. The oral cavity patch according to any one of claims 1 to 6, wherein the adhesive layer comprises, as an adhesive agent, at least one selected from the group consisting of a carboxyalkyl cellulose, a carboxyalkyl cellulose salt, alginic acid, an alginate salt, poly(N-vinyl lactam), polyvinyl alcohol, hydroxypropyl cellulose, carboxyvinyl polymer, pullulan, and pregelatinized starch.

8. The oral cavity patch according to any one of claims 1 to 7, wherein the adhesive layer comprises a carboxyalkyl cellulose or a carboxyalkyl cellulose salt as an adhesive agent.

9. The oral cavity patch according to any one of claims 1 to 8, wherein the adhesive layer comprises, as an adhesive agent, a carboxyalkyl cellulose or a carboxyalkyl cellulose salt, and poly(N-vinyl lactam).

10. The oral cavity patch according to any one of claims 1 to 8, wherein the adhesive layer comprises, as an adhesive agent, a carboxyalkyl cellulose or a carboxyalkyl cellulose salt, and polyvinylpyrrolidone.

11. The oral cavity patch according to any one of claims 8 to 10, wherein the carboxyalkyl cellulose salt is sodium carboxymethyl cellulose.

12. The oral cavity patch according to any one of claims 1 to 11, wherein the oral cavity patch is a tablet.

## INTERNATIONAL SEARCH REPORT

| International application No. |
|---|
| PCT/JP2014/064415 |

A. CLASSIFICATION OF SUBJECT MATTER
*A61K47/38*(2006.01)i, *A61K9/20*(2006.01)i, *A61K47/12*(2006.01)i, *A61K47/26*
(2006.01)i, *A61K47/32*(2006.01)i, *A61K47/34*(2006.01)i, *A61K47/36*(2006.01)i,
*A61K47/46*(2006.01)i
According to International Patent Classification (IPC) or to both national classification and IPC

B. FIELDS SEARCHED

Minimum documentation searched (classification system followed by classification symbols)
A61K47/38, A61K9/20, A61K47/12, A61K47/26, A61K47/32, A61K47/34,
A61K47/36, A61K47/46

Documentation searched other than minimum documentation to the extent that such documents are included in the fields searched
Jitsuyo Shinan Koho          1922–1996   Jitsuyo Shinan Toroku Koho   1996–2014
Kokai Jitsuyo Shinan Koho    1971–2014   Toroku Jitsuyo Shinan Koho   1994–2014

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 09-235220 A  (Sanwa Kagaku Kenkyusho Co., Ltd.), 09 September 1997 (09.09.1997), claims; examples & US 5914118 A          & EP 781546 A1 | 1–12 |
| X | JP 2003-504236 A  (Gruenenthal GmbH), 04 February 2003 (04.02.2003), claims; examples & US 2002/0142036 A1    & EP 1194133 A | 1–12 |
| X | JP 10-298063 A  (Permatec Technologie AG), 10 November 1998 (10.11.1998), claims; examples & US 6242004 B1          & EP 873750 A1 | 1–12 |

| ☒ | Further documents are listed in the continuation of Box C. | ☐ See patent family annex. |

\* Special categories of cited documents:
"A" document defining the general state of the art which is not considered    to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 June, 2014 (23.06.14) | 15 July, 2014 (15.07.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2014/064415 |

| C (Continuation). | DOCUMENTS CONSIDERED TO BE RELEVANT | |
|---|---|---|
| Category* | Citation of document, with indication, where appropriate, of the relevant passages | Relevant to claim No. |
| X | JP 11-513982 A (Ceratech Inc.), 30 November 1999 (30.11.1999), claims; examples & US 5766620 A & US 5863555 A & EP 859606 A & WO 1997/015296 A1 | 1-12 |
| X | JP 63-60924 A (Teikoku Seiyaku Co., Ltd.), 17 March 1988 (17.03.1988), claims; examples & US 5196202 A & US 4889720 A & EP 262422 A1 | 1-12 |
| X | JP 11-513983 A (Ceratech Inc.), 30 November 1999 (30.11.1999), claims; examples & US 5849322 A & EP 857063 A & WO 1997/015297 A1 | 1-12 |
| X | JP 58-79916 A (Sand AG.), 13 May 1983 (13.05.1983), claims; examples & GB 2108841 A & DE 3237945 A & FR 2514642 A | 1-12 |
| X | JP 2000-178185 A (Lion Corp.), 27 June 2000 (27.06.2000), claims; examples (Family: none) | 1-12 |
| X | JP 2000-63268 A (Lion Corp.), 29 February 2000 (29.02.2000), claims; examples (Family: none) | 1-12 |
| X | JP 55-62012 A (Teijin Ltd.), 10 May 1980 (10.05.1980), claims; examples & US 4292299 A & EP 20777 A1 & WO 1980/000916 A1 | 1-12 |
| X | JP 2010-138123 A (Kyukyu Pharmaceutical Co., Ltd.), 24 June 2010 (24.06.2010), claims; examples; paragraph [0015] (Family: none) | 1-12 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S5562012 B
- JP H3209326 B
- JP 2006096728 A

**Non-patent literature cited in the description**

- Hypromellose. The Japanese Pharmacopoeia
- Viscosity Determination, Method II Viscosity measurement by rotational viscometer. General Test, Process and Apparatus in The Japanese Pharmacopoeia